# EUROPEAN PATENT SPECIFICATION

(11) **EP 3 739 036 B1**
(45) Date of publication and mention of the grant of the patent: **08.02.2023**
(21) Application number: 18906036.1
(22) Date of filing: 17.12.2018
(51) Int. Cl.: G06V 10/40, G06V 10/778, G06V 20/69, C12M 1/00, C12M 1/32, C12M 1/34, G01N 35/00

(54) **BIOLOGICAL SUBJECT TRANSFER DEVICE**
ÜBERTRAGUNGSVORRICHTUNG FÜR BIOLOGISCHE OBJEKTE
DISPOSITIF DE TRANSFERT DE SUJET BIOLOGIQUE

(30) Priority: 15.02.2018 JP 2018024716
(43) Date of publication of application: 18.11.2020
(73) Proprietor: Yamaha Hatsudoki Kabushiki Kaisha, Iwata-shi, Shizuoka-ken 438-8501 (JP)
(72) Inventor: SAKAMOTO, Masaru, Iwata-shi, Shizuoka-ken 438-8501 (JP)
(74) Representative: Müller-Boré & Partner Patentanwälte PartG mbB
(86) International application number: PCT/JP2018/046336
(87) International publication number: WO 2019/159524

(56) References cited:
- EP-A1- 3 733 832
- WO-A1-2017/110005
- JP-A- 2009 063 508
- JP-A- 2014 235 494
- LONG X ET AL: "Automatic detection of unstained viable cells in bright field images using a support vector machine with an improved training procedure", COMPUTERS IN BIOLOGY AND MEDICINE, NEW YORK, NY, US, vol. 36, no. 4, April 2006 (2006-04), pages 339-362, XP028025049, ISSN: 0010-4825, DOI: 10.1016/J.COMPBIOMED.2004.12.002 [retrieved on 2006-04-01]
- CONAGHAN JOE ET AL: "Improving embryo selection using a computer-automated time-lapse image analysis test plus day 3 morphology: results from a prospective multicenter trial", FERTILITY AND STERILITY, ELSEVIER, AMSTERDAM, NL, vol. 100, no. 2, 28 May 2013 (2013-05-28), page 412, XP028685721, ISSN: 0015-0282, DOI: 10.1016/J.FERTNSTERT.2013.04.021

## Description

### Technical Field

The present invention relates to a biological subject transfer device, which comprises an image capturing system including an image capturing device that captures an image of a biological subject such as, for example, cells or cell clusters.

### Background Art

For example, in medical and biological research applications, image capturing for selecting cells or cell clusters (example of a biological subject; sometimes simply referred to as "cell") may be performed. For example, the work of capturing images of cells accommodated in a transfer-source first container with an image capturing device, selecting desired cells based on the obtained images, and sucking the selected cells with a tip and transferring the cells to a transfer-destination second container may be performed (for example, Patent Literature 1).

Specific examples of cell selection to be performed on the first container side include a method of depending on an operator's manual selection, a method of setting in advance a selection reference value about the size, shape, and the like of the cell, and the like. The former method is a method of depending on skill of an individual by which the operator observes a captured image of the cell and makes a quality determination based on experience of the operator. The latter method is a method of determining a parameter related to the size and shape of a cell by image processing on the captured image of the cell, and automatically making a quality determination based on whether the parameter satisfies the selection reference value.

If the cell is selected as the desired cell on the transfer-source first container side and the cell transferred to the transfer-destination second container is observed again on the second container side as described above, there may be cases where the cell is not the intended cell. That is, if the cell is observed again after the intervention of "work" of cell transfer, the cell actually may not be the intended cell. A similar situation may occur due to the intervention of various types of work. Therefore, there is a problem that work efficiency of inspections, tests, and the like performed on the second container side is reduced.

### Citation List

### Patent Literature

Patent Literature 1: WO 2015/087371 A.

Patent Literature: EP 3 733 832 A1 is directed at an image capturing system that includes: a camera unit (5) that captures an image of a cell (C); a display unit (74) that displays the image including the captured cell; an input unit (75) that receives input from an operator regarding a selection operation on the cell (C); an analyzing unit (731) that analyzes the image corresponding to the cell (C) to which the selection operation is given and extracts a feature amount of the cell; and a specifying unit (732) that specifies a recommended cell to be presented to the operator.

Non-Patent Literature: LONG X ET AL: "Automatic detection of unstained viable cells in bright field images using a support vector machine with an improved training procedure", COMPUTERS IN BIOLOGY AND MEDICINE, NEW YORK, NY, US, vol. 36, no. 4, April 2006 (2006-04), pages 339-362, XP028025049, ISSN: 0010-4825, DOI: 10.1016/J.COMPBIOMED.2004.12.002 is directed at detection of a viable cell using a bright field in combination with fluorescence imaging as a training procedure.

Patent Literature: WO 2017/110005 A1 is directed at a pick-up method for target objects, whereby a tip (6) having a tip opening (6H) is inserted into and suctions inside a well (3) of a well plate (10) supporting a target object (C), said method comprising: step in which the well plate (10) is captured by an imaging means (5) and a well frame (35A) for the well (3) is recognized by an image processing means (73) on the basis of this image; a step in which central coordinates in an upper surface view of the well (3) are found by a calculation means (74) on the basis of the well frame (35A); and a step in which the tip (6) is lowered towards the well (3), in a state in which the tip (6) is positioned above an axial line for the central coordinates in a space above the well (3). Non-Patent Literature: CONAGHAN JOE ET AL: "Improving embryo selection using a computer-automated time-lapse image analysis test plus day 3 morphology: results from a prospective multicenter trial", FERTILITY AND STERILITY, ELSEVIER, AMSTERDAM, NL, vol. 100, no. 2, 28 May 2013 (2013-05-28), page 412, XP028685721, ISSN: 0015-0282, DOI: 10.1016/J. FE RTNSTE RT. 2013.04.021 is directed at monitoring of developing embryos and selection of an embryo.

### Summary of Invention

### Problems to be Solved by the Invention

An object of the present invention is to provide an image capturing system that enables accurate selection of the biological subject required by the operator, and a biological subject transfer device using the image capturing system.

The present invention is defined by the features described in the independent claim.

Additional embodiments are defined in the dependent claims.

### Brief Description of Drawings

FIG. 1 is a diagram schematically showing a configuration example of a cell transfer device to which an image capturing system according to an embodiment of the present invention is applied.
FIG. 2A is a top view of a dish included in a selection container used in the cell transfer device, and FIG. 2B is a cross-sectional view taken along line IIB-IIB of FIG. 2A.
FIG. 3A is a perspective view of a microplate used in the cell transfer device, and FIG. 3B is a longitudinal cross-sectional view of FIG. 3A.
FIG. 4 is a diagram for describing a concept of feedback of cell selection in the present embodiment.
FIG. 5 is a diagram showing a feedback example of cell selection in a first embodiment in which work is a cell transfer.
FIGS. 6A and 6B are diagrams showing a feedback example of cell selection in a second embodiment in which work is a cell selection by an operator.
FIGS. 7A and 7B are diagrams showing a feedback example of cell selection in a third embodiment in which work is a change in an image capturing condition.
FIGS. 8A and 8B are diagrams showing a feedback example of cell selection in a modification of the third embodiment in which work is a change in an image capturing condition.
FIGS. 9A to 9D are diagrams showing a feedback example of cell selection in a fourth embodiment in which work is dispensing of a reagent.
FIG. 10 is a diagram showing a feedback example of cell selection in a fifth embodiment in which work is work of waiting for an elapse of a test time.
FIG. 11 is a block diagram of the cell transfer device according to the embodiment of the present invention.
FIG. 12 is a flowchart of a cell transfer operation using the image capturing system.

### Description of Embodiments

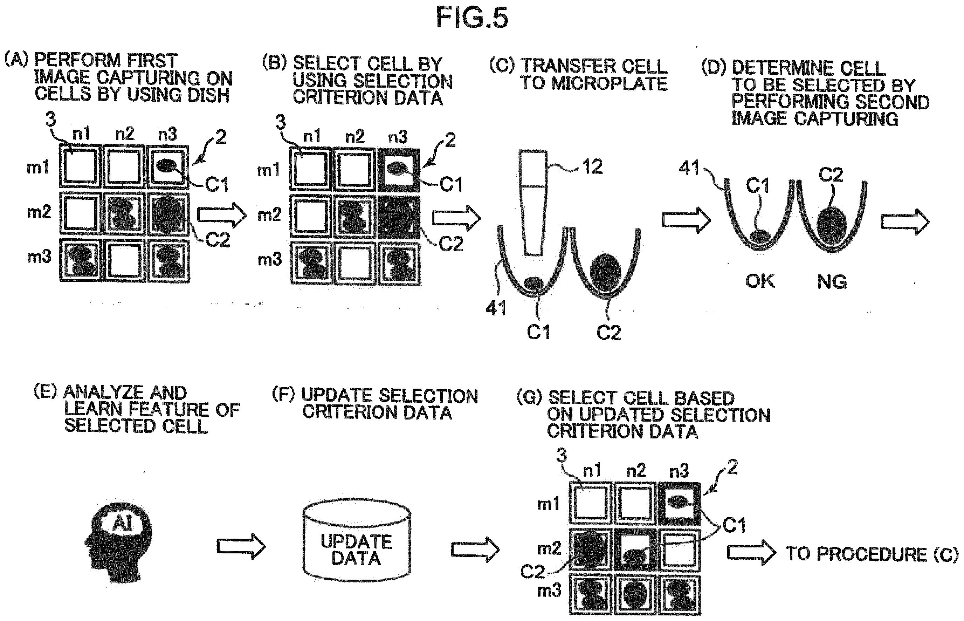

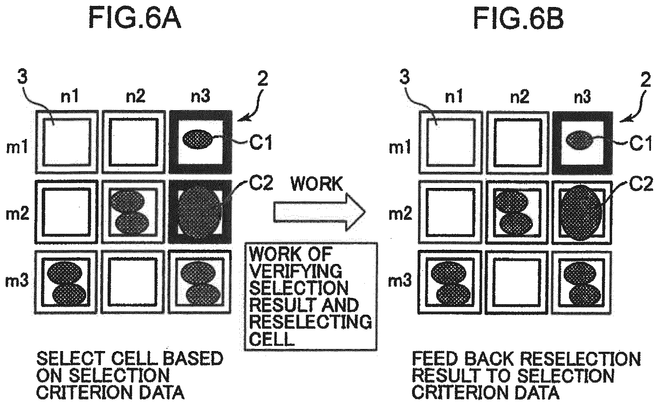

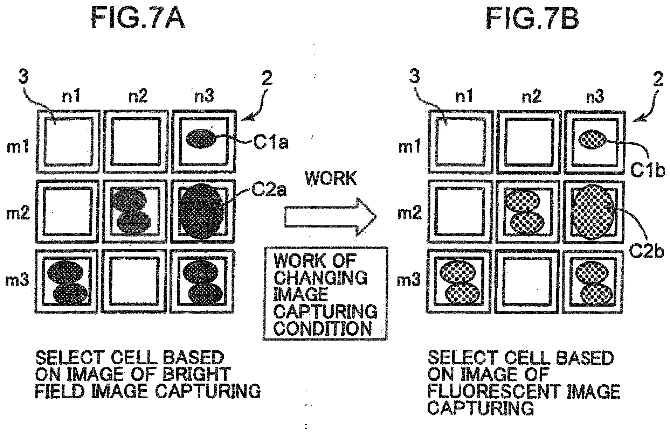

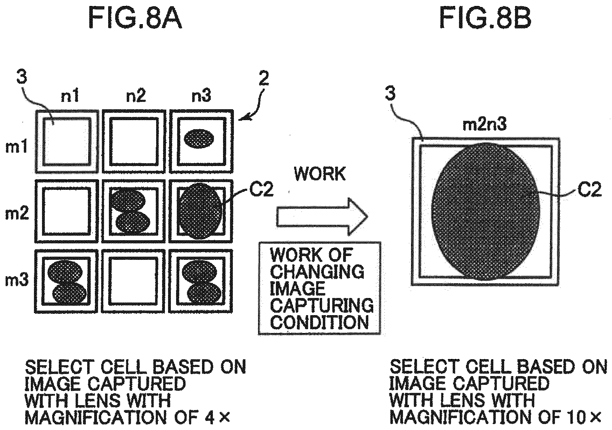

Embodiments of the present invention will be described in detail below with reference to the drawings. The following embodiments are not encompassed by the wording of the claims but are considered as useful for understanding the invention: the first embodiment shown in FIG. 5, the second embodiment shown in FIGS. 6A and 6B, the third embodiment shown in FIGS. 7A and 7B and the modification of the third embodiment shown in FIGS. 8A and 8B.

An image capturing system according to the present invention can capture images of a wide variety of biological subjects. In the present invention, as the biological subject to be captured, a cell of biological origin can be typically illustrated. Examples of the cell of biological origin here include a single cell (cell) such as a blood cell and single cell, tissue fragment such as Histoculture and CTOS, cell aggregation cluster such as spheroids and organoids, individuals such as zebrafish, nematodes, fertilized eggs, and 2D or 3D colony. In addition, tissues, microorganisms, small species, and the like can be illustrated as the biological subject. The embodiment described below show an example in which the biological subject is cells or a cell aggregation cluster formed by aggregating several to several hundred thousand cells (hereinafter, collectively referred to simply as "cell C").

### [Overall structure of cell transfer device]

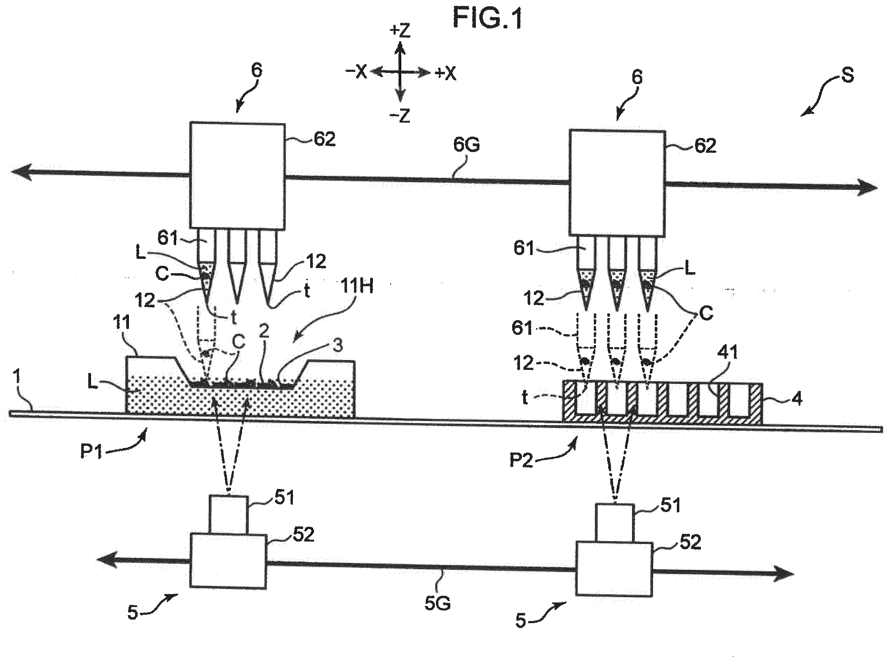

FIG. 1 is a diagram schematically showing an overall configuration of a cell transfer device S to which an image capturing system according to an embodiment of the present invention is applied. Here, the cell transfer device S that transfers a cell C between two containers (dish 2 and microplate 4) is illustrated.

The cell transfer device S includes a translucent base 1 having an upper surface, which is a horizontal mounting surface, a camera unit 5 (image capturing device) placed below the base 1, and a head unit 6 (head device) placed above the base 1. A selection container 11 including the dish 2 (first container) is mounted at a first mounting position P1 of the base 1, and the microplate 4 (second container) is mounted at a second mounting position P2. The head unit 6 includes a plurality of heads 61 to which tips 12 that each suck and discharge the cell C are attached, the heads 61 capable of moving in a Z direction (up-and-down direction). The camera unit 5 and the head unit 6 are movable in the X direction (horizontal direction) and the direction perpendicular to the plane of FIG. 1 (Y direction). The dish 2 and the microplate 4 are mounted on an upper surface of the base 1 within a movable range of the head unit 6.

Roughly, the cell transfer device S is a device in which each of the plurality of tips 12 sucks the cell C individually from the dish 2 of the selection container 11 holding a large number of cells C, and transfers the cell C to the microplate 4, and the plurality of tips 12 simultaneously discharge the cells C to wells 41 of the microplate 4. Before the suction of the cells C, the cells C held in the dish 2 are captured by the camera unit 5 (first image capturing), and selection work of selecting good quality cells C to be transferred to the microplate 4 is performed. After the transfer of the cells C, the cells C accommodated in the microplate 4 are captured (second image capturing) by the camera unit 5, and the cells C are observed for the purpose of verifying the validity of the selection work.

Each part of the cell transfer device S will be described below. The base 1 is a rectangular flat plate having predetermined rigidity, and part or all of which is formed of a translucent material. The preferred base 1 is a glass plate. The base 1 is formed of a translucent material such as a glass plate, thereby allowing the camera unit 5 placed below the base 1 to capture the selection container 11, the dish 2, and the microplate 4 placed on an upper surface of the base 1 through the base 1.

The selection container 11 is a container that is a transfer source of the cells C, stores a culture medium L, and holds the dish 2 for cell selection in a state of being immersed in the culture medium L. The dish 2 is a plate that holds the cells C, and has a plurality of holding recesses 3 that can individually accommodate and hold the cells C on an upper surface. The culture medium L is not particularly limited as long as the culture medium L does not deteriorate the properties of the cells C, and can be appropriately selected depending on the type of cell C.

The selection container 11 includes a rectangular upper opening 11H on the upper surface side. The upper opening 11H is an opening for injecting the cells C and picking up the selected cells C. The dish 2 is placed below the upper opening 11H. The selection container 11 and the dish 2 made of a translucent resin material or glass is used. This is to allow the camera unit 5 placed below the selection container 11 to observe the cells C supported in the dish 2.

A plurality of cells C dispersed in a cell culture solution is injected into the selection container 11 from a dispensing tip (not shown). The dispensing tip sucks the cell culture solution together with the cells C from a container that stores the cell culture solution containing the large amount of cells C, and holds the cell culture solution in the dispensing tip. Thereafter, the dispensing tip is moved to an upper air position of the selection container 11 to access the upper surface of the dish 2 through the upper opening 11H. Then, with a tip opening of the dispensing tip immersed in the culture medium L of the selection container 11, the cells C held in the dispensing tip are discharged onto the dish 2 together with the cell culture solution.

The microplate 4 is a container serving as a transfer destination for the cells C, and includes a plurality of wells 41 in which the cells C are discharged. The wells 41 are each a bottomed hole opened on an upper surface of the microplate 4. One well 41 accommodates a required number of (usually one) cells C together with the culture medium L. The microplate 4 made of a translucent resin material or glass is used. This is to allow the camera unit 5 placed below the microplate 4 to observe the cells C supported in the well 41.

The camera unit 5 captures an image of the cells C held in the selection container 11 or the microplate 4 from the lower surface side thereof, and includes a lens unit 51 and a camera body 52. The lens unit 51 is an object lens used in an optical microscope, and includes a lens group that forms a light image with a predetermined magnification and a lens barrel that accommodates the lens group. The camera body 52 includes an image capturing element such as a CCD image sensor. The lens unit 51 forms a light image of an image capturing target on a light receiving surface of the image capturing element. The camera unit 5 is movable in the X and Y directions below the base 1 along a guide rail 5G extending in the left-right direction parallel to the base 1. In addition, the lens unit 51 is movable in the Z direction for a focusing operation.

The head unit 6 is provided for picking the cell C selected as a transfer target from the dish 2 serving as the first container that accommodates a plurality of cells C, and performing transfer work (predetermined work) of transferring the cell C to the microplate 4 serving as the second container. The head unit 6 includes a plurality of heads 61 and a head body 62 to which the heads 61 are assembled. At the tip of each head 61, the tip 12 that sucks (pickup) and discharges the cells C is attached. The head body 62 holds the heads 61 so as to be raised and lowered in the +Z and -Z directions, and is movable in the +X and -X directions along a guide rail 6G. Note that the head body 62 is also movable in the Y direction.

### [Details of dish and microplate]

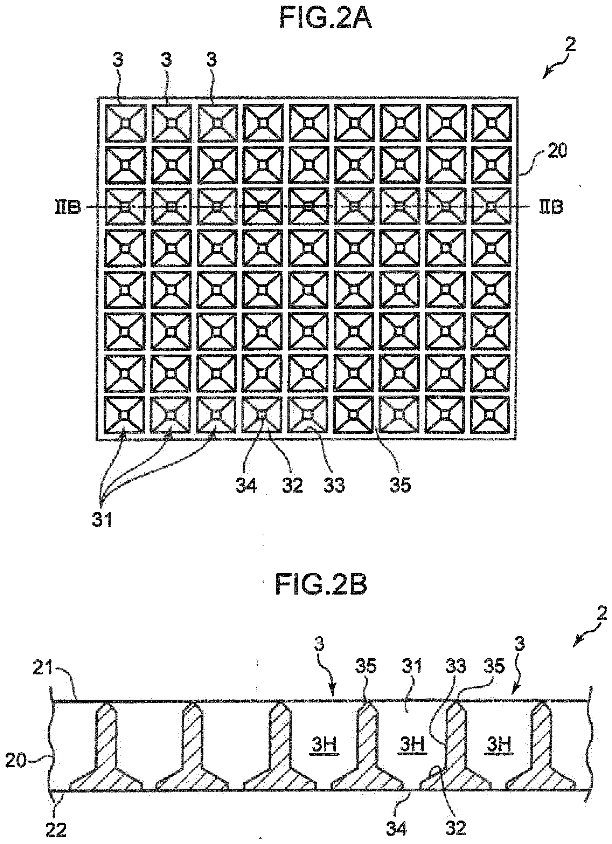

First, detailed structure of the dish 2, which is a transfer-source container, will be described. FIG. 2A is a top view of the dish 2, and FIG. 2B is a cross-sectional view taken along line IIB-IIB of FIG. 2A. The dish 2 includes a dish body 20 and a plurality of holding recesses 3 formed in the dish body 20. The dish body 20 includes a flat plate-shaped member having a predetermined thickness and includes an upper surface 21 and a lower surface 22. Each holding recess 3 includes an opening portion 31 serving as an opening that receives the cell C on the upper surface 21 side. The dish 2 is immersed in the culture medium L in the selection container 11. In detail, the dish 2 is held in the selection container 11 with a space between the lower surface 22 and a bottom plate of the selection container 11 while the upper surface 21 of the dish body 20 is immersed in the culture medium L in the selection container 11 (see FIG. 1).

Each of the holding recesses 3 includes an opening portion 31, a bottom portion 32, a cylindrical wall surface 33, a hole portion 34, and a boundary portion 35. The present embodiment shows an example in which the holding recesses 3 that are square in top view are arranged in a matrix. As shown in FIG. 2B, the plurality of holding recesses 3 is arranged in a matrix at a predetermined recess arrangement pitch.

The opening portion 31 is a square opening provided on the upper surface 21, and has a size that allows entrance of a tip opening portion t of the tip 12 for selection. The bottom portion 32 is positioned inside the dish body 20 and near the lower surface 22. The bottom portion 32 is an inclined surface that gently inclines downward toward the center (center of the square). The cylindrical wall surface 33 is a wall surface extending vertically downward from the opening portion 31 toward the bottom portion 32. The hole portion 34 is a through hole vertically penetrating between the center of the bottom portion 32 and the lower surface 22. The boundary portion 35 is a portion that is positioned on the upper surface 21 and serves as an opening edge of each holding recess 3, and is a ridge line that partitions the holding recesses 3 from each other.

The bottom portion 32 and the cylindrical wall surface 33 of each holding recess 3 define an accommodation space 3H that accommodates the cell C. It is generally intended that one cell C is accommodated in the accommodation space 3H. The hole portion 34 is provided to allow a small cell or impurities having a size other than a desired size to escape from the accommodation space 3H. Therefore, the size of the hole portion 34 is selected such that the cell C having the desired size cannot pass through but a small cell and impurities other than the desired size can pass through. Accordingly, the cell C to be selected is trapped in the holding recess 3, while impurities and the like fall from the hole portion 34 to the bottom plate of the selection container 11.

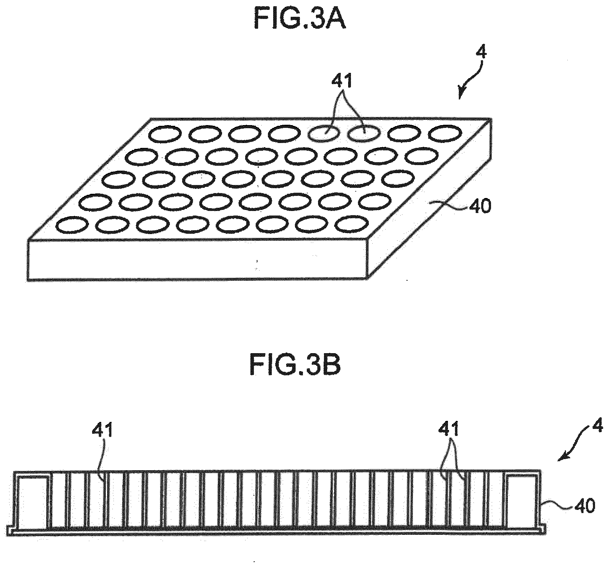

Next, the microplate 4, which is a transfer-destination container, will be described. FIG. 3A is a perspective view of the microplate 4, and FIG. 3B is a longitudinal cross-sectional view of the microplate 4. The microplate 4 includes a plate body 40 and a plurality of wells 41 arranged in a matrix in the plate body 40. Since the tip opening portion t of the tip 12 enters the well 41 when the cell C is discharged, each well 41 has an opening diameter that allows the entry of the tip 12 with a margin.

There is a reference size for commercially available microplates. The reference microplate has a predetermined longitudinal × lateral size (longitudinal 85.48 mm × lateral 126 mm) and includes a predetermined number of wells. The general number of wells is 24 × 16 (384 wells), and the wells are arranged in a matrix at a predetermined pitch. FIG. 3B is a cross-sectional view of the microplate 4 with 384 wells. As shown in the FIG. 3B, 24 wells 41 are arranged at an equal well pitch in the longitudinal direction of the microplate 4 (16 wells in the lateral direction).

### [Outline of cell selection work]

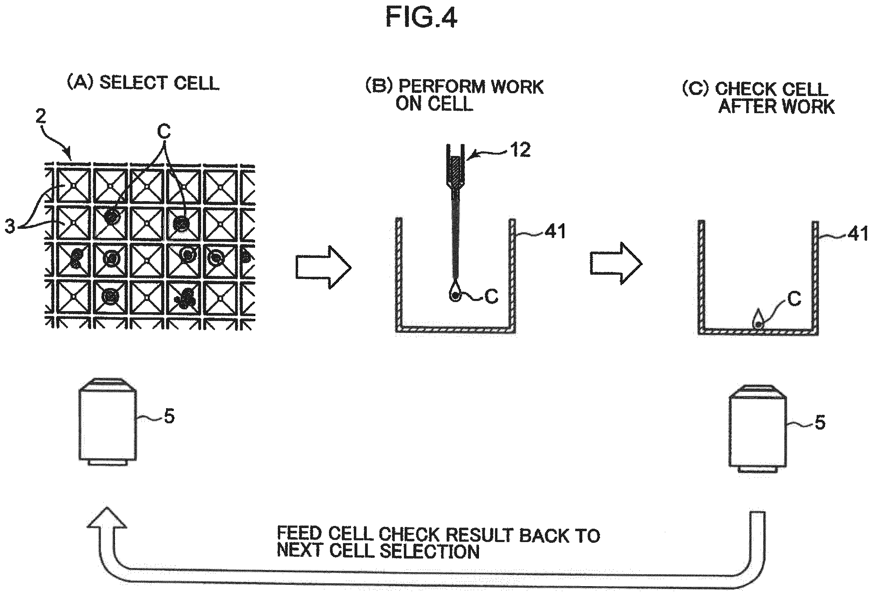

FIG. 4 is a diagram for describing the outline of the cell selection work (steps (A) to (C)) in the present embodiment. First, in step (A), the camera unit 5 captures an image of the dish 2 (first image capturing), and acquires a first image of the cells C supported in the holding recesses 3 of the dish 2. In this first image, the cells C before predetermined work is performed, in the present embodiment, the cells C before the transfer work of the cell C is performed are captured. Then, based on a predetermined selection criterion from the first image, a first determination is made to determine which of the cells C captured in the image is to be selected as the transfer target.

The selection criterion is, for example, data of a manual selection criterion that is inherently determined by each operator who performs the cell selection work based on experience or the like, or data of a selection criterion determined in advance regarding the size, shape, or the like of the cell. When relying on the latter, a feature amount of the cells C captured in the first image is obtained by image processing. In this case, the first determination is a determination as to whether the obtained feature amount satisfies the selection criterion data.

In step (B), work on the cell C is performed. In the present embodiment, the work comprises individually picking the cell C selected as the transfer target in the first determination from the holding recess 3 of the dish 2 by using the tip 12, and transferring the cell C to one well 41 of the microplate 4. In addition, work of waiting for an elapse of a test time for the cell C is performed.

In step (C), the camera unit 5 captures an image of the microplate 4 to which the cell C has been transferred by the transfer work in step (B) (second image capturing), and acquires a second image of the cell C held in the well 41. In the second image, the cell C that has undergone the predetermined work is captured. In the second image, a second determination is made to check validity of each cell C transferred to the well 41. The second determination is determination as to which cell C is to be selected from among the cells C captured in the image, for example, as a target of the work on the cell C in the next stage, for example, work of adding a reagent, inspection, observation, or the like.

In this way, the selection of the cell C is determined in the two stages of the first determination and the second determination for the following reason. First, it can be cited that the work performed between the first and second determinations, that is, the transfer work may cause deformation, deterioration, collapse, or the like of the cell C. For example, an easily deformable cell C may be deformed when sucked by the tip 12, or what looks like one cell C may be decomposed into a plurality of cells.

Next, it can be cited that an erroneous determination is made in the first determination. The first determination is made based on the image of the cell C supported in the holding recess 3. That is, the first determination is made based on the feature amount such as the shape, color tone, or the like of the cell C appearing in the first image acquired through a bottom surface of the selection container 11 and the bottom portion 32 of the holding recess 3. Therefore, an error factor may intervene in a transmitted light image of the cell C, and also, only the lower half surface of the cell C can be observed. Therefore, if the cell C is observed again based on the second image acquired after the work, the cell C may actually be found to be defective. In some cases, it may be possible to recognize that the cell C is defective only after the second image capturing is performed under an image capturing condition different from the first image capturing. In addition, the cell C may be found to be defective by acquiring the second image after work of applying some treatment to the cell C or after an aging period. It is requested to make the second determination for such a reason.

If a determination result different from a result of the first determination is obtained in the second determination of step (C), the discrepancy is fed back to the first determination in the next procedure (A). Specifically, if the cell C of discrepancy (error) that has been determined differently between the first and second determinations is generated, the feature amount of the cell C (error) is obtained, for example, from the second image. Then, in the subsequent determination processing, selection criterion data on which the first determination relies is updated with reference to the feature amount of the cell C (error) such that the first determination and the second determination on the cell C (error) come close to each other, that is, they become identical to each other. By repeating such an update (learning), only the cell C determined to be "selected" in the second determination will be selected in the first determination as well, and the first determination and the second determination are gradually homogenized. In a short time, the cell C that is truly necessary can be selected only by the first determination, and the second determination can be omitted, enabling improvement in the work efficiency. Examples of feedback for cell selection are specifically shown below for each work type.

### [First Embodiment]

FIG. 5 is a diagram schematically showing procedures (A) to (G) of feedback of cell selection in a first embodiment in which the work is the cell transfer outlined above. In the first procedure (A), the first image capturing is performed in which the camera unit 5 captures an image of the dish 2. By this first image capturing, the first image of the cells C supported in the holding recesses 3 of the dish 2 is acquired. The lens unit 51 of the camera unit 5 has an angle of view that can simultaneously capture an image of a plurality of cells C. The procedure (A) shows an example in which an image of the holding recesses 3 of a 3 × 3 (m1 to m3 × n1 to n3) matrix is captured by one image capturing operation. The example here shows a state in which one cell C is supported in each of the holding recesses 3 of mln3 and m2n3, two cells C are supported in each of the holding recesses 3 of m2n2, m3n1, and m3n3, and no cell C is supported in other holding recesses 3.

In the subsequent procedure (B), based on the first image acquired in the procedure (A), the first determination is made about which cell C is to be transferred. As described above, the selection criterion data is used for the first determination. The feature amount of the cell C held in each holding recess 3 is extracted by analyzing the first image. Examples of the feature amount include an amount of cell C obtained from the number, area, estimated volume, and the like of the cell C, color and appearance of the cell C, light intensity when the cell C is fluorescent, and the like. Analysis results of the cell C in each holding recess 3 are digitized. The selection criterion data is, for example, a parameter that defines the range of cell C to be selected. In the first determination, it is determined whether the feature amount of each cell C belongs to the range of the parameter, and the cell C that belongs to the range is selected as the transfer target. Here, an example is shown in which the cell C1 of m1n3 and the cell C2 of m2n3 are selected as the transfer target (highlighted by black frames in FIG. 5. The same applies hereinafter.).

In the next procedure (C), as predetermined work, the cells C1 and C2 selected as the transfer target are transferred to each well 41 of the microplate 4 by the tip 12. When the transfer is completed, as shown in the procedure (D), the second image capturing is performed to capture the image of the microplate 4 holding the transferred cells C1 and C2 by the camera unit 5. Then, based on the second image obtained by the second image capturing, the second determination is made as to whether to select the cells C1 and C2 for subsequent work. The second determination may be made by either of manual selection by the operator or automatic selection using the feature amount. Here, an example is shown in which the cell C 1 is selected (OK) and the cell C2 is not selected (NG) in the second determination. Note that as the feature amount, information such as the number, amount, color, appearance, light intensity, and the like of the cell C accommodated in the well 41 can be used. In particular, the number of cells C is information that can be most easily identified, including the case where the number is 0, and thus it is preferable that at least the number of cells C be included in the feature amount.

In the subsequent procedure (E), the feature amount of the cell C1 selected in the second determination or the cell C2 not selected in the second determination is extracted based on the second image. Then, as shown in the procedure (F), the extracted feature amount is fed back (updated) to the selection criterion data to be used in the first determination. In this way, in the first determination based on the previous selection criterion data, the cell C2 has been determined to be "selected", but in the subsequent first determination, the cell C2 is determined to be "not selected".

The procedure (G) shows an example of cell selection after the feedback. That is, in the first determination after the feedback, the cell C1 or a cell C in a similar category is selected, but the cell C2 or a cell C in a similar category is not selected. Thereafter, a transition is made to the procedure (C), the selected cell C1 is transferred to each well 41, and the second determination of the procedure (D) is made similarly. In this case, since only the cell C1 is transferred to the well 41, the cell C1 will be selected in the second determination. That is, in the selection of the cell C, no discrepancy occurs between the first determination and the second determination.

### [Second Embodiment]

FIGS. 6A and 6B are diagrams showing a feedback example of cell selection in a second embodiment in which work is a cell selection by an operator. Here, an example is shown in which the operator manually reselects validity of a cell C selected in the first determination based on the selection criterion data described above in the dish 2. That is, before the transfer work of the cell C described in the first embodiment, the operator performs the work of the manual selection operation that also serves as the second determination on the cell C automatically selected based on the selection criterion data. Then, the result of the cell C by the manual selection operation is fed back to the first determination.

FIG. 6A shows a result of the first determination made on the first image acquired by the first image capturing based on the selection criterion data at a certain time point. Here, an example that is not covered by the claims is shown in which the cell C1 of m1n3 and the cell C2 of m2n3 are selected in the first determination. The operator makes the second determination (reselection operation) as to whether these cells C1 and C2 are appropriate as the selection target by the manual selection operation. Note that since the image of the dish 2 is referred to in the second determination, the first image acquired previously is used. That is, in the present embodiment that is not covered by the claims, the second image is not newly acquired, and the first image is used as the second image.

FIG. 6B is a diagram showing a result of the second determination by the operator. Here, an example is shown in which the cell C1 is selected but the cell C2 is not selected in the second determination. Thereafter, in a similar manner to the procedures (E) and (F) of the first embodiment, the feature amount of the cell C1 or the cell C2 is extracted, and the extracted feature amount is fed back to the selection criterion data used for the first determination. With this operation, in the subsequent first determination, it is determined that the cell C2 is "not selected".

### [Third Embodiment]

FIGS. 7A and 7B are diagrams showing a feedback example of cell selection in a third embodiment in which work is a change in an image capturing condition. As described above, in the embodiments of the present invention, the first determination and the second determination are made based on a two-dimensional image of a cell C. In this case, by changing the image capturing condition, it may be possible to clearly make a quality determination of the cell C that is difficult to make with a captured image under the same image capturing condition. In view of this point, in the third embodiment, first image capturing is performed to acquire a first image under a predetermined image capturing condition and the first determination is made, and the image capturing condition is changed to acquire a second image in subsequent second image capturing and the second determination is made. That is, work in the third embodiment is work of changing the image capturing condition in the first image capturing. Then, a selection result of the cell C by the second determination is fed back to the first determination.

FIG. 7A is the first image of the cells C captured by the first image capturing, showing an image obtained by bright field image capturing. That is, FIG. 7A is an image acquired by a camera unit 5 under the condition of "bright field image capturing". In the first image, the first determination is made based on the selection criterion data at that time. Thereafter, the work of changing the image capturing condition is performed. Examples of changeable image capturing conditions include an angle of view, image capturing angle, magnification, light exposure, nature of illumination light, amount of light, lens type, and the like. Needless to say, a change in the camera unit 5 used is included.

FIG. 7B is the second image of the cells C acquired by the second image capturing after the image capturing condition is changed, and in this case, shows an image obtained by fluorescent image capturing. That is, FIG. 7B is an image acquired by the camera unit 5 under the condition of "fluorescent image capturing". The fluorescent image capturing is performed, for example, by adding a fluorescent agent or using fluorescent illumination. In the second image, for example, the operator makes the second determination as to which cell C is to be selected by a manual selection operation. A selection result of the cell C by the second determination is fed back to the first determination.

When the cell C1a of m1n3 and the cell C2a of m2n3 observed in the first image of bright field image capturing are observed in the second image of fluorescent image capturing, the cells C1a and C2a are observed as cells C1b and C2b having different light images, respectively. Here, a defect of the cell C overlooked by the bright field image capturing may be observed by the fluorescent image capturing. That is, if based on an image acquired by changing a method of viewing the cell C, more accurate selection determination can be made in some cases. The defect may actually appear as a feature amount in the first image of bright field image capturing. Therefore, by feeding back the result of the second determination based on the second image of fluorescent image capturing to the selection criterion data, more accurate first determination can be made.

FIGS. 8A and 8B are diagrams showing a modification of the third embodiment. Work of changing the image capturing condition in this modification is work of changing image capturing magnification of a lens unit 51 of the camera unit 5. FIG. 8A is the first image of the cells C captured by the first image capturing, and shows, for example, an image captured with a lens with a magnification of 4×. Meanwhile, FIG. 8B is the second image of the cell C acquired by the second image capturing after the image capturing magnification is changed, and shows, for example, an image of the cell C2 of m2n3 captured with a lens with a magnification of 10×.

In some cases, the determination of necessity of selection (second determination) performed on the cell C2 based on the enlarged second image of FIG. 8B may allow more accurate determination than the determination of necessity of selection (first determination) performed on the cell C2 based on the first image of FIG. 8A. For example, a defect that is unable to be identified in the 4× image may be observed in the 10× image. Therefore, by feeding back the result of the second determination based on the second image with the higher magnification to the selection criterion data, the first determination can be made more accurately.

### [Fourth Embodiment]

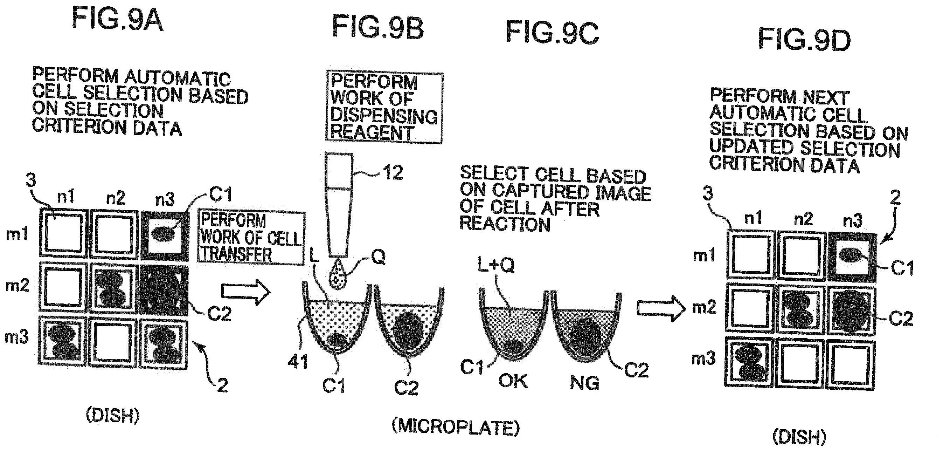

FIGS. 9A to 9D are diagrams showing a feedback example of cell selection in a fourth embodiment in which work is dispensing of a reagent. A selected cell C is subjected to a sensitivity test to a reactive test substance such as a reagent or a growth agent in many cases. It is possible that a cell C selected as a good quality cell C suitable for the test is a cell C unsuitable for the sensitivity test. In view of this point, in the fourth embodiment, after the first determination, work of adding a reactive test substance to the selected cell C is performed. The second image capturing is performed on the cell C after the work to acquire the second image, and a quality determination (second determination) is made on the cell C. Then, a selection result of the cell C by the second determination is fed back to the first determination.

FIG. 9A shows a result of the first determination made on the first image captured by the first image capturing based on the selection criterion data at a certain time. Here, an example is shown in which the cell C1 of m1n3 and the cell C2 of m2n3 are selected in the first determination. As first work, work of transferring the selected cells C1 and C2 to respective wells 41 of a microplate 4 by a tip 12 is performed. The work up to this point is the same as in the first embodiment described above.

Subsequently, as shown in FIG. 9B, as second work, work of dispensing a reagent Q by using the tip 12 is performed on each well 41 accommodating the transferred cell C1 or C2 and the culture medium L. After a predetermined time has elapsed after the dispensing of the reagent Q is finished, as shown in FIG. 9C, the second image capturing is performed by the camera unit 5 that captures the image of the microplate 4 holding the transferred cells C1 and C2. With this operation, the image of the cells C1 and C2 after the reaction to the reagent Q is acquired as the second image. In this second image, for example, the operator makes the second determination to select whether the cells C1 and C2 are appropriate as targets for the sensitivity test by a manual selection operation. Here, an example is shown in which the cell C1 is selected (OK) and the cell C2 is not selected (NG) in the second determination.

Then, as shown in FIG. 9D, the selection result of the cells C1 and C2 by the second determination is fed back to the first determination. That is, the feature amount of the cell C1 or C2 is extracted, and the extracted feature amount is fed back (updated) to the selection criterion data used for the first determination. With this operation, in the subsequent first determination, it is determined that the cell C2 is "not selected". Therefore, the cell C is selected before the addition work of the reagent Q so as to follow the selection result of the cell C after the addition work of the reagent Q.

### [Fifth Embodiment]

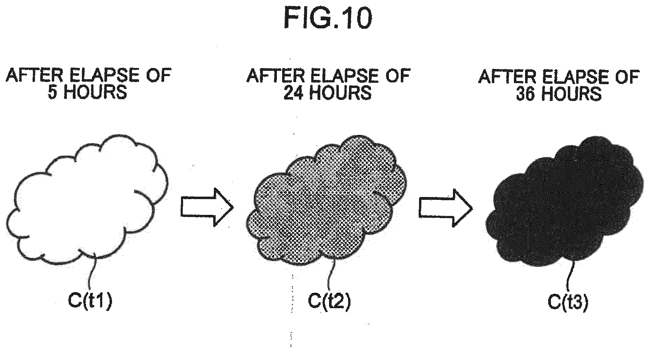

FIG. 10 is a diagram showing a feedback example of cell selection in a fifth embodiment in which work is work of waiting for an elapse of a test time. Even if a cell C is left in a culture medium L without particularly adding a reagent Q or the like, the shape and properties of the cell C may change. For example, growth, death, division, discoloration, and the like of the cell C can be illustrated. FIG. 10 shows an example in which the color of the cell C changes as time elapses, and simply illustrates the state of cells C (t1), C (t2), and C (t3) after 5 hours, 24 hours, and 36 hours elapse, respectively. Therefore, waiting for an elapse of a predetermined test time for the cell C can also be the "work" for the cell C.

The fifth embodiment can be implemented, for example, by replacing the reagent dispensing work in FIG. 9B of the fourth embodiment with the work of waiting for an elapse of a test time. In this case, the second image capturing and cell selection (second determination) of FIG. 9C are performed after the elapse of a predetermined test time after the cells C1 and C2 are transferred to the wells 41. Then, a result of the second determination is fed back to the first determination,

### [Electric configuration of cell transfer device]

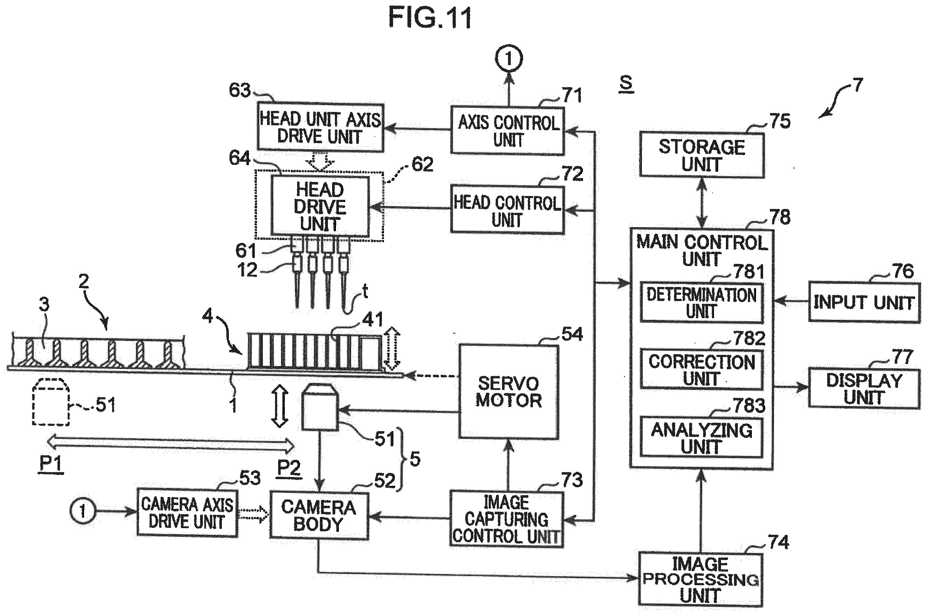

FIG. 11 is a block diagram showing an electrical configuration of the cell transfer device S. The cell transfer device S includes a control unit 7 that controls movement of the head unit 6, raising and lowering of the heads 61 and the tips 12, suction and discharge operations of the cell C, movement and image capturing operations of the camera unit 5, and the like. In addition, the cell transfer device S includes a camera axis drive unit 53 serving as a mechanism for horizontally moving the camera unit 5, a servo motor 54 serving as a driving source for moving the lens unit 51 up and down, a head unit axis drive unit 63 serving as a mechanism for horizontally moving the head unit 6, and a head drive unit 64 serving as a mechanism for raising and lowering the head 61 and a mechanism for performing suction and discharge operations.

The camera axis drive unit 53 includes a drive motor that horizontally moves the camera unit 5 along the guide rail 5G (FIG. 1). The camera axis drive unit 53 moves the camera unit 5 between the first mounting position P1 directly under the dish 2 and the second mounting position P2 directly under the microplate 4.

The servo motor 54 rotates forward or backward to move the lens unit 51 in an up-and-down direction with a predetermined resolution via a power transmission mechanism (not shown). By this movement, the focus position of the lens unit 51 is adjusted to the cells C accommodated in the holding recesses 3 of the dish 2 or the wells 41 of the microplate 4. Note that as shown by the dotted line in FIG. 11, instead of the lens unit 51, the selection container 11 or the microplate 4 itself or the base 1 that is a stage on which the selection container 11 and the microplate 4 are mounted may be moved up and down by the servo motor 54.

The head unit axis drive unit 63 includes a drive motor that moves the head unit 6 (head body 62) along the guide rail 6G. The head drive unit 64 includes a motor serving as a power source that raises and lowers the head 61 with respect to the head body 62, and a mechanism serving as a power source that generates suction force and discharge force at the tip opening portion t of the tip 12.

The control unit 7 includes a microcomputer or the like, and functions to include an axis control unit 71, a head control unit 72, an image capturing control unit 73, an image processing unit 74, a storage unit 75, and a main control unit 78 by executing a predetermined program. Furthermore, an input unit 76 that inputs various information items to the control unit 7 and a display unit 77 that displays various information items are included. The input unit 76 functions as a terminal that receives input regarding the cell C selection operation from the operator. The display unit 77 functions as a monitor that displays the first image, the second image, and the like captured by the camera unit 5.

The axis control unit 71 controls the operation of the head unit axis drive unit 63. That is, the axis control unit 71 controls the head unit axis drive unit 63 to move the head unit 6 to a predetermined target position in the horizontal direction. Movement of the head 61 (tip 12) between the selection container 11 and the microplate 4, positioning in the vertically upper position with respect to the holding recess 3 of the dish 2, and positioning in the vertically upper position with respect to the well 41 of the microplate 4 serving as a discharge target are implemented by the control of the head unit axis drive unit 63 by the axis control unit 71.

The head control unit 72 raises and lowers the head 61 to be controlled toward a predetermined target position by controlling the head drive unit 64. Also, the head control unit 72 generates suction force or discharge force at the tip opening portion t of the tip 12 at predetermined timing by controlling the suction mechanism corresponding to the head 61 to be controlled.

The image capturing control unit 73 controls the operation of moving the camera unit 5 along the guide rail 5G by controlling the camera axis drive unit 53. Also, the image capturing control unit 73 controls the image capturing operation of the dish 2 or the microplate 4 by the camera unit 5, such as, for example, the exposure amount and shutter timing. Furthermore, the image capturing control unit 73 gives control pulses for moving the lens unit 51 in an up-and-down direction at a predetermined pitch (for example, tens of µm pitch) to the servo motor 54 for the focusing operation.

The image processing unit 74 performs image processing such as edge detection processing and pattern recognition processing with feature amount extraction on image data acquired by the camera body 52. Based on the image of the dish 2 after the cells C are dispensed, the image processing unit 74 performs processing of recognizing the presence and number of the cells C on the holding recesses 3 of the dish 2 on the image, processing of acquiring the XY coordinates of each cell C, processing of acquiring condition information such as an outer contour, size such as an area and volume, shape, color tone, and the like of individual cell C. Similarly, based on the image of the wells 41 to which the cells C are transferred, the image processing unit 74 performs processing of recognizing the number, an amount such as total area and total volume, fluorescence intensity, and the like of the cells C accommodated in the wells 41.

The storage unit 75 stores various setting values, data, programs and the like in the cell transfer device S. In addition, the storage unit 75 stores data regarding the selection criterion of the cell C to be used for the first determination. As described above, the selection criterion data is updated according to the cell selection result of the second determination made after the predetermined work is performed on the cell C.

The main control unit 78 comprehensively controls the operations of the camera unit 5 and the head unit 6. The main control unit 78 controls the camera unit 5 and the head unit 6 through the axis control unit 71, the head control unit 72, and the image capturing control unit 73 to capture the image of the dish 2 on which the cells C are scattered at the first mounting position P1 (FIG. 1) where the selection container 11 is mounted, perform picking to cause the tip 12 attached to the head 61 to suck the cells C selected as the transfer target, and transfer the cells C to the microplate 4. In such comprehensive control, the main control unit 78 makes the first determination for automatically selecting the cell C to be transferred from the dish 2, and the second determination for determining whether to select the cell C transferred to the microplate 4.

The main control unit 78 functionally includes a determination unit 781, a correction unit 782, and an analyzing unit 783 for the first and second determinations. The determination unit 781 performs processing of the first determination of selecting the cell C to be transferred to the microplate 4 from the first image of the dish 2 supporting the cells C captured by the camera unit 5 (first image capturing). With reference to the selection criterion data stored in the storage unit 75, the determination unit 781 performs the first determination based on whether the feature amount of each cell C acquired by the image processing of the first image by the image processing unit 74 agrees with the selection criterion data.

Also, the determination unit 781 performs processing of the second determination of selecting the cell C to be used for subsequent work from the second image of the microplate 4 after the transfer work of the cell C, the second image being captured by the camera unit 5 (second image capturing). The second determination can rely on the operator's selection operation of the cell C received by the input unit 76. Alternatively, the selection criterion data for the second determination may be stored in the storage unit 75, and the determination unit 781 may make a determination automatically.

When the first determination and the second determination have different determination results about a certain cell C, the correction unit 782 performs processing of updating the selection criterion data stored in the storage unit 75 such that the first determination and the second determination about the cell C become identical in the subsequent determination. Specifically, the correction unit 782 reflects the feature amount of the cell C in which discrepancy has occurred in the selection criterion data. That is, when the cell C is determined as "not selected" in the second determination, the selection criterion data is updated to prevent the feature amount of the cell C from being included in the selection criterion data. On the other hand, when the cell C that is determined as "not selected" in the first determination is determined as "selected" in the second determination, conversely, the selection criterion data is updated such that the feature amount of the cell C is included in the selection criterion data.

By analyzing the image of the cell C specified by the image processing unit 74 in the first image or the second image described above, the analyzing unit 783 performs processing of extracting the feature amount of the cell C. Examples of the feature amount to be extracted include the shape, number, area, estimated volume, color, appearance, light intensity, or the like of the cell C. The analyzing unit 783 digitizes these feature amounts, and the determination unit 781 and the correction unit 782 perform predetermined processing by using this numerical value. For example, for the cell C selected or not selected in the second determination, the analyzing unit 783 extracts the feature amount of the cell C based on the image acquired in the second image capturing. The correction unit 782 updates the selection criterion data by using the numerical value of the extracted feature amount.

In a stage where determination is made that learning of the selection criterion data has progressed by the update processing of the correction unit 782, the determination unit 781 can execute an automatic determination mode in which the result of the first determination is used in the second determination. This is because, by repeating the update (learning) of the selection criterion data, the first determination and the second determination are gradually homogenized before long, and it becomes possible to select the truly necessary cell C only by the first determination.

### [Flow of cell transfer operation]

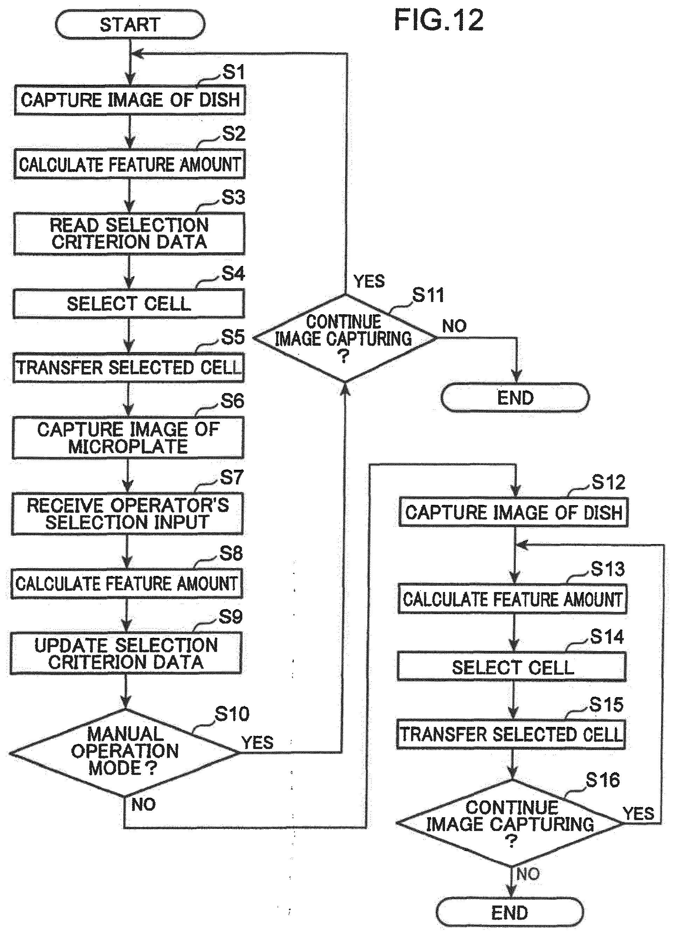

Subsequently, the cell transfer operation using the image capturing system of the present embodiment shown in FIG. 11 will be described with reference to the flowchart shown in FIG. 12. When the processing starts, the main control unit 78 causes the camera unit 5 to capture an image of the dish 2 (first image capturing). A cell suspension has been dispensed in the dish 2 in advance. The camera unit 5 captures an image of the cells C accommodated in the holding recesses 3 of the dish 2 (step S1).

Next, the image processing unit 74 performs image processing of acquiring image data of the dish 2 acquired by the image capturing from the camera body 52, and specifying the cells C included in the image. The image processing data is sent to the analyzing unit 783 of the main control unit 78. The analyzing unit 783 performs processing of determining the feature amount of the specified cell C such as the shape, number, area, estimated volume, color, appearance, light intensity, and the like of the cell C (step S2).

Subsequently, the determination unit 781 reads the selection criterion data of the cell C from the storage unit 75 (step S3), and with reference to the selection criterion data, the determination unit 781 performs the first determination of determining which of the cells C supported in the dish 2 is to be selected as the transfer target (step S4).

When the cell C to be transferred is determined, the main control unit 78 performs cell transfer work of transferring the cell C from the dish 2 serving as the first container to the microplate 4 serving as the second container (step S5). Specifically, the head control unit 72 controls the head drive unit 64 to cause the tip 12 attached to the head 61 to pick the cell C supported in the holding recess 3 of the dish 2. The axis control unit 71 controls the head unit axis drive unit 63 to move the head unit 6 to the position above the microplate 4. Furthermore, the head control unit 72 controls the head drive unit 64 to discharge the cell C sucked by the tip 12 into the predetermined well 41.

After discharge of the cell C into the well 41 is finished, the main control unit 78 causes the camera unit 5 to capture an image of the microplate 4 (second image capturing) (step S6). The image acquired by this image capturing is displayed on the display unit 77. The operator visually recognizes the display unit 77 and determines which cell C is to be selected from among the cells C transferred to respective wells 41 of the microplate 4 for subsequent work. The determination result is received by the input unit 76. The determination unit 781 treats instruction information input to the input unit 76 as the second determination of selecting the cell C after predetermined work (step S7).

If there is a cell C for which different determinations are made between the first determination of step S4 and the second determination of step S7, the feature amount of the cell C is calculated by the analyzing unit 783 (step S8). Then, the correction unit 782 updates the selection criterion data for the first determination stored in the storage unit 75 based on the feature amount extracted by the analyzing unit 783 (step S10). Note that when there is no discrepancy between the first determination and the second determination, steps S8 and S9 are skipped.

Subsequently, the main control unit 78 determines whether the operation mode set in the cell transfer device S is a manual operation mode (step S10). The manual operation mode is a mode of performing the image capturing in step S6 and the input reception in step S7 after the transfer work of the cell C, and is a mode performed in a stage where the selection criterion data of the storage unit 75 is not sufficiently learned.

When the operation mode is set as the manual operation mode (YES in step S10), the main control unit 78 confirms whether the next image capturing of the dish 2 (first image capturing) is scheduled (step S11). When the first image capturing is scheduled (YES in step S11), the main control unit 78 returns to step S1 to perform the next first image capturing. In the next routine, in step S4, the selection criterion data updated in step S9 is used.

On the other hand, when the automatic determination mode is set instead of the manual operation mode in step S10 (NO in step S10), the main control unit 78 performs the operation from which steps S6 to S9 described above are omitted. The automatic determination mode is a mode performed when reaching a stage where determination is made that the learning of the selection criterion data has progressed by the update processing of the correction unit 782, and is a mode in which the result of the first determination is treated as the result of the second determination.

In this case, the main control unit 78 causes the camera unit 5 to capture an image of the dish 2 supporting the cells C (first image capturing) (step S12), and the feature amount of the cells C in the acquired image is calculated by the analyzing unit 783 (step S13). Subsequently, the determination unit 781 makes the first determination of selecting the cell C to be transferred with reference to the selection criterion data stored in the storage unit 75 (step S14). Then, the main control unit 78 transfers the selected cell C from the dish 2 to the microplate 4 (step S15). As described above, since the second determination is omitted in the automatic determination mode, the work in the next stage, for example, addition of a reagent or the like is performed on all the transferred cells C.

Thereafter, it is confirmed whether to continue the image capturing of the cell C (step S16). When the image capturing is continued (YES in step S16), returning to step S13, the camera unit 5 performs the next image capturing operation on the dish 2. On the other hand, when there is no cell C to be captured (NO in step S16), the process ends.

With the image capturing system according to the present embodiment described above, regarding the selection of the cell C, in the image acquired by the first image capturing performed before the predetermined work on the cell C, the first determination selected by the determination unit 781 based on the selection criterion data is compared with the second determination based on the image acquired by the second image capturing acquired after the predetermined work is performed. Then, if there is a discrepancy between the two, the correction unit 782 updates the selection criterion data such that the first determination and the second determination become identical. Therefore, the selection criterion data is gradually corrected so as to agree with the second determination made after predetermined work. Therefore, it is possible to increase the probability of determining to be "selected" in the second determination, and increase the work efficiency of subsequent inspections and tests on the cell C.

## Claims

1. A biological subject transfer device (S) comprising
an image capturing system (S) comprising:
an image capturing device (5) capable of performing first image capturing to capture an image of a biological subject (C) accommodated in a first container (2) and second image capturing to capture an image of the biological subject (C) after predetermined work is performed, the predetermined work being work of transferring by the biological subject transfer device (S) the biological subject (C) selected as a transfer target from the first container (2) accommodating a plurality of biological subjects (C) to a second container (4);
a storage unit (75) configured to store data regarding the selection criterion;
a head device (61) configured to perform transfer work of picking the biological subject (C) selected as the transfer target from the first container (2) accommodating the plurality of biological subjects (C) and transferring the biological subject (C) to the second container (4) as the predetermined work;
a determination unit (781) configured to make a first determination to determine whether to select the biological subject (C) based on a predetermined selection criterion from the image acquired by the first image capturing,
**characterized in that**
the determination unit (781) is additionally configured to make a second determination to determine whether to select the biological subject (C) from the image acquired by the second image capturing;
a correction unit (782) is configured to update the selection criterion data stored in the storage unit (75) such that the first determination and the second determination about the biological subject (C) become identical in a subsequent determination when the first determination and the second determination have different determination results; and
the predetermined work further includes work of waiting for an elapse of a predetermined test time after having transferred the biological subject (C) to the second container (4) before the second image capturing is performed by a camera unit (5) after the first image capturing.

2. The biological subject transfer device (S) according to claim 1, wherein the image capturing system (S) further comprises an input unit (76) configured to receive input from an operator regarding a selection operation on the biological subject (C),
wherein the predetermined work further includes the selection operation received by the input unit (76), and wherein the second determination relies on the operator's selection operation of the biological subject (C) received by the input unit (76).

3. The biological subject transfer device (S) according to claim 1, wherein the image capturing system (S) further comprises an analyzing unit (783) configured to extract, regarding the biological subject selected or the biological subject not selected in the second determination, a feature amount of the biological (C) subject based on the image captured by the second image capturing,
wherein the correction unit (782) updates the data regarding the selection criterion based on the feature amount extracted by the analyzing unit (783).

4. The biological subject transfer device (S) according to claim 3, wherein
the second container (4) includes a plurality of wells (41) that accommodate the biological subjects (C), and
the feature amount includes information on a number of the biological subjects (C) accommodated in each of the wells (41).

5. The biological subject transfer device (S) according to any one of claims 1 to 4, wherein in a stage in which determination is made that learning of the data regarding the selection criterion has progressed due to the update by the correction unit (782), the determination unit (781) has an automatic determination mode in which a result of the first determination is used in the second determination.

## Patentansprüche

1. Transfervorrichtung für ein biologisches Subjekt (S), aufweisend:
ein Bilderfassungssystem (S):
eine Bilderfassungsvorrichtung (5), die in der Lage ist, erste Bilderfassung durchzuführen, um ein Bild eines biologischen Subjekts (C) zu erfassen, das in einem ersten Behälter (2) aufgenommen ist, sowie eine zweite Bilderfassung durchzuführen, um ein Bild des biologischen Subjekts (C) aufzunehmen, nachdem vorbestimmte Arbeiten durchgeführt wurden, wobei es sich bei den vorbestimmten Arbeiten um Arbeiten für den Transfer, durch die Transfervorrichtung (S) für ein biologisches Subjekt (C), das als Transferziel ausgewählt wurde, von dem ersten Behälter (2), der eine Vielzahl biologischer Subjekte (C) aufnimmt, in den zweiten Behälter (4) handelt;
eine Speichereinheit (75), die konfiguriert ist, Daten in Bezug auf das Auswahlkriterium zu speichern;
eine Kopfvorrichtung (61), die konfiguriert ist, Transferarbeiten des Aufgreifens des biologischen Subjekts (C), das als Transferziel ausgewählt wurde, aus dem ersten Container (2), der die Vielzahl biologischer Subjekte (C) aufnimmt, sowie des Transfers des biologischen Subjekts (C) in den zweiten Behälter (4) als die vorbestimmten Arbeiten durchzuführen;
eine Bestimmungseinheit (781), die konfiguriert ist, eine erste Bestimmung vorzunehmen, um basierend auf einem vorbestimmten Auswahlkriterium aus dem Bild, das mittels der ersten Bilderfassung erlangt wurde, zu bestimmen, ob das biologische Subjekt (C) ausgewählt werden soll,
**dadurch gekennzeichnet, dass**
die Bestimmungseinheit (781) zusätzlich konfiguriert ist, eine zweite Bestimmung vorzunehmen, um zu bestimmen, ob aus dem Bild, das mittels der ersten Bilderfassung erlangt wurde, das biologische Subjekt (C) ausgewählt werden soll;
eine Korrektureinheit (782) konfiguriert ist, die Auswahlkriteriumsdaten, die in der Speichereinheit (75) gespeichert sind, derart zu aktualisieren, dass die erste Bestimmung und die zweite Bestimmung bezüglich des biologischen Subjekts (C) in einer darauffolgenden Bestimmung identisch werden, wenn die erste Bestimmung und die zweite Bestimmung unterschiedliche Bestimmungsergebnisse aufweisen; und
die vorbestimmten Arbeiten ferner Arbeiten des Abwartens eines Verstreichens einer vorbestimmten Testdauer umfassen, nachdem das biologische Subjekt (C) in den zweiten Behälter (4) transferiert wurde, bevor nach der ersten Bilderfassung die zweite Bilderfassung durch eine Kameraeinheit (5) durchgeführt wird.

2. Transfervorrichtung für ein biologisches Subjekt (S) nach Anspruch 1, wobei das Bilderfassungssystem (S) ferner eine Eingabeeinheit (76) aufweist, die konfiguriert ist, eine Eingabe eines Bedieners in Bezug auf den Auswahlvorgang zu dem biologischen Subjekt (C) zu empfangen,
wobei die vorbestimmten Arbeiten den durch die Eingabeeinheit (76) empfangenen Auswahlvorgang umfassen, und wobei die zweite Bestimmung auf dem Auswahlvorgang des Bedieners für das biologische Subjekt (C), der von der Eingabeeinheit (76) empfangen wird, beruht.

3. Transfervorrichtung für ein biologisches Subjekt (S) nach Anspruch 1, wobei das Bilderfassungssystem (S) ferner eine Analyseeinheit (783) aufweist, die konfiguriert ist, in Bezug auf das bei der zweiten Bestimmung ausgewählte biologische Subjekt, oder das bei der zweiten Bestimmung nicht ausgewählte Subjekt, basierend auf dem mittels der zweiten Bilderfassung erfassten Bild eine Merkmalsmenge des biologischen Subjekts (c) zu extrahieren,
wobei die Korrektureinheit (782) die Daten in Bezug auf das Auswahlkriterium basierend auf der von der Analyseeinheit (83) extrahierten Merkmalsmenge aktualisiert.

4. Transfervorrichtung für ein biologisches Subjekt (S) nach Anspruch 3, wobei
der zweite Behälter (4) eine Vielzahl von Titern (41) aufweist, die die biologischen Subjekte (C) aufnehmen, und
die Merkmalsmenge Informationen über eine Anzahl der biologischen Subjekte (C) aufweist, die in jedem der Titer (41) aufgenommen sind.

5. Transfervorrichtung für ein biologisches Subjekt (S) nach einem der Ansprüche 1 bis 4, wobei in einer Phase, in der die Bestimmung erfolgt, dass das Lernen der Daten in Bezug auf das Auswahlkriterium aufgrund der Aktualisierung durch die Korrektureinheit (782) Fortschritte gemacht hat, die Bestimmungseinheit (781) einen automatischen Bestimmungsmodus aufweist, in welchem ein Ergebnis der ersten Bestimmung bei der zweiten Bestimmung verwendet wird.

## Revendications

1. Dispositif de transfert de sujet biologique (S) comprenant
un système de capture d'image (S) comprenant :
un dispositif de capture d'image (5) capable d'effectuer une première capture d'image pour capturer une image d'un sujet biologique (C) logé dans un premier contenant (2) et une deuxième capture d'image pour capturer une image du sujet biologique (C) après que l'activité prédéterminée a été effectuée, l'activité prédéterminée étant une activité de transfert, par le dispositif de transfert du sujet biologique (S), du sujet biologique (C) sélectionné comme une cible à transférer depuis le premier contenant (2) logeant une pluralité de sujets biologiques (C) vers un deuxième contenant (4) ;
une unité de stockage (75) configurée pour stocker des données concernant le critère de sélection ;
un dispositif de tête (61) configuré pour effectuer une activité de transfert de prélèvement du sujet biologique (C) choisi comme la cible à transférer parmi le premier contenant (2) logeant la pluralité de sujets biologiques (C) et de transfert du sujet biologique (C) au deuxième contenant (4) comme l'activité prédéterminée ;
une unité de détermination (781) configurée pour réaliser une première détermination pour déterminer s'il faut sélectionner le sujet biologique (C) sur la base d'un critère de sélection prédéterminé d'après l'image acquise par la première capture d'image,
**caractérisé en ce que**
l'unité de détermination (781) est configurée en supplément pour réaliser une deuxième détermination pour déterminer s'il faut sélectionner le sujet biologique (C) parmi l'image acquise par la deuxième capture d'image ;
une unité de correction (782) est configurée pour mettre à jour les données de critère de sélection stockées dans l'unité de stockage (75) de telle sorte que la première détermination et la deuxième détermination concernant le sujet biologique (C) deviennent identiques dans une détermination ultérieure quand la première détermination et la deuxième détermination ont des résultats de détermination différents ; et
l'activité de détermination comporte en outre une activité d'attente pendant un intervalle de temps d'un test prédéterminé après avoir transféré le sujet biologique (C) au deuxième contenant (4) avant que la deuxième capture d'image ne soit effectuée par une unité de caméra (5) après la première capture d'image.

2. Dispositif de transfert de sujet biologique (S) selon la revendication 1, dans lequel le système de capture d'image (S) comprend en outre une unité d'entrée (76) configurée pour recevoir des entrées d'un opérateur concernant une opération de sélection sur le sujet biologique (C),
dans lequel l'activité prédéterminée comporte en outre l'opération de sélection reçue par l'unité d'entrée (76), et dans lequel la deuxième détermination repose sur l'opération de sélection de l'opérateur du sujet biologique (C) reçue par l'unité d'entrée (76).

3. Dispositif de transfert de sujet biologique (S) selon la revendication 1, dans lequel le système de capture d'image (S) comprend en outre une unité d'analyse (783) configurée pour extraire, concernant le sujet biologique sélectionnée ou le sujet biologique non sélectionné dans la deuxième détermination, une quantité de caractéristiques du sujet biologique (C) sur la base de l'image capturée par la deuxième capture d'image,
dans lequel l'unité de correction (782) met à jour les données concernant le critère de sélection sur la base de la quantité de caractéristiques extraites par l'unité d'analyse (783).

4. Dispositif de transfert de sujet biologique (S) selon la revendication 3, dans lequel le deuxième contenant (4) comporte une pluralité de puits (41) qui logent les sujets biologiques (C), et
la quantité de caractéristiques comporte des informations sur un nombre de sujets biologiques (C) logés dans chacun des puits (41).

5. Dispositif de transfert de sujet biologique (S) selon l'une quelconque des revendications 1 à 4, dans lequel dans une phase, dans laquelle la détermination est réalisée selon laquelle l'apprentissage des données concernant le critère de sélection a avancé du fait de la mise à jour par l'unité de correction (782), l'unité de détermination (781) présente un mode de détermination automatique, dans lequel un résultat de la première détermination est utilisé dans la deuxième détermination.
